# EUROPEAN PATENT SPECIFICATION

(11) **EP 4 084 748 B1**
(45) Date of publication and mention of the grant of the patent: **24.06.2026**
(21) Application number: 20842246.9
(22) Date of filing: 30.12.2020
(51) Int. Cl.: A61F 9/00, A61M 16/16, A61M 16/00, A61M 11/02, A61M 11/00, A61H 35/02, A61F 9/02, A61B 3/10, A61H 33/14

(54) **PROCEDURE AND DEVICE TO PRODUCE A REFLEX TEAR SECRETION AND A KIT FOR THE MEASUREMENT OF THE MAGNITUDE OF THE EVOKED TEAR FLOW**
VERFAHREN UND VORRICHTUNG ZUM PRODUZIEREN EINER REFLEX-TRÄNENSEKRETION UND EIN KIT ZUR MESSUNG DER GRÖSSE DES EVOZIERTEN TRÄNENFLUSSES
PROCÉDÉ ET DISPOSITIF POUR PRODUIRE UNE SÉCRÉTION LACRYMALE RÉFLEXE ET KIT POUR LA MESURE DE L'AMPLITUDE DU FLUX DE LARMES ÉVOQUÉ

(30) Priority: 31.12.2019 ES 201931176
(43) Date of publication of application: 09.11.2022
(73) Proprietor: Universidad Miguel Hernández de Elche (UMH), 03202 Alicante (ES); Consejo Superior De Investigaciones Científicas, 28006 Madrid (ES)
(72) Inventor: BELMONTE MARTÍNEZ, Carlos, 03550 SAN JUAN DE ALICANTE (ES); GALLAR MARTÍNEZ, Juana, 03550 SAN JUAN DE ALICANTE (ES); ACOSTA BOJ, María Carmen, 03110 MUCHAMIEL (ES); MERINO SUÁREZ, María Luisa, 03110 MUCHAMIEL (ES)
(74) Representative: Sugrañes, S.L.P.
(86) International application number: PCT/EP2020/088057
(87) International publication number: WO 2021/136820

(56) References cited:
- EP-A1- 3 583 889
- WO-A1-2017/156050
- WO-A1-94/12104
- WO-A2-2006/082588
- M. CARMEN ACOSTA ET AL: "Tear Secretion Induced by Selective Stimulation of Corneal and Conjunctival Sensory Nerve Fibers", INVESTIGATIVE OPTHALMOLOGY & VISUAL SCIENCE, vol. 45, no. 7, 1 July 2004 (2004-07-01), pages 2333 - 2336, XP055771372, ISSN: 1552-5783, DOI: 10.1167/iovs.03-1366

## Description

### Technical section of the invention

The present invention relates to a procedure and a device for evoking maximum reflex tear secretion, by applying a chemical stimulus onto the corneal surface of a human or an animal. Measurement of the tear volume secreted in response to stimulation can be used for the diagnosis of ophthalmic and autoimmune diseases, especially dry eye disease.

The invention also relates to a kit for measuring the magnitude of the tear flow generated by said device, which comprises, in addition to the device, a set of modified measuring strips for carrying out the Schirmer test, covering high secretion volumes.

### Background of the invention

Keratoconjunctivitis sicca or Dry Eye Disease, also known by the acronym DED, is currently one of the pathologies that concentrates the greatest research effort in the field of ocular pathology. In general, it has been well established that the fundamental causes of DED are either the reduction in tear production, or the excess evaporation of the tear film as a consequence of alterations in its composition, and that there is a complex regulatory mechanism in charge of maintaining the homeostasis of the ocular surface, whose alteration plays a decisive role in the pathology of the disease. However, many of the functional characteristics of the ocular surface moisture regulation system and its disorders in pathological circumstances are still unknown.

The ocular surface maintains an adequate degree of humidity thanks to the tear film that covers it, formed by a muco-aqueous layer in contact with the epithelium and an external lipid layer. Tears are continuously secreted by specialized glands, distributing homogeneously throughout the ocular surface thanks to the regular closure of the eyelids. On the other hand, evaporation, mainly dependent on environmental factors, tends to continuously reduce the aqueous component of the tear film. Despite the variable intrinsic (changes in the composition and volume of the tear) or environmental (humidity, wind, temperature) factors that tend to modify the tear film, it maintains, under normal conditions, a constant composition and volume thanks to complex neural regulation mechanisms, which adjust the flow of tears and the blinking rhythm to the changing environmental conditions to which the eyes of land mammals are continuously exposed.

The cornea has numerous sensitive nerve endings that respond to chemical, mechanical, and thermal stimuli that act on the ocular surface. The stimulation of these terminals, belonging to polymodal nociceptor, mechano-nociceptor and cold thermoreceptor sensory neuron types (M. Carmen Acosta et al: "Tear Secretion Induced by Selective Stimulation of Corneal and Conjunctival Sensory Nerve Fibers", investigative Ophthalmology & Visual Science, vol. 45, no. 7, 1 July 2004 (2004-07-01), pages 2333-2336, XP055771372), activates a reflex arc that has the purpose of re-establishing the homeostatic balance of the ocular surface and also protecting it from dryness, external aggressions or foreign bodies, through a reflex increase in tear secretion, which is maximal when the stimulus activates all corneal polymodal nociceptor nerve fibers. These findings have been confirmed by direct *in vivo* and *in vitro* electrophysiological recordings of the activity of sensory nerve endings on the surface of the eye. In humans, it is not possible to directly record the nervous activity that, acting reflexively on the lacrimal glands, modulates the volume of tear secretion. To know the maximum secretory response of tear glands, this must be measured using indirect techniques. Determining the maximum tear volume that can be secreted by DED patients is critical to assessing the actual ability of their lacrimal glands to counteract the desiccation of the ocular surface. One of the most used techniques to diagnose DED is the Schirmer test, which measures basal tear secretion. From a clinical point of view, basal tear secretion is defined as the volume of tears produced during a unit of time.

The Schirmer test consists of inserting into the lower outer third of the eye a 35mm long strip of absorbent paper, which is maintained for a specified time, usually 5 min., keeping the eyes closed. The tear film covering the bulbar conjunctiva in contact with the upper part of the paper strip soaks with the tear fluid, gradually advancing along its length. Once the test time has elapsed, the strip of paper is removed and the length of the strip moistened by the tear is measured, expressing the basal tear secretion flow in millimeters. A normal measurement in a healthy patient corresponds to a wetted length above 10 mm on the paper scale, in 5 minutes. Medical personnel classify DED patients into different categories and level of severity according to their basal tear secretion value.

In order to minimize discomfort to the patient due to the irritation that the contact of the paper strip with the conjunctival surface may produce, in the Schirmer test with anesthesia, ophthalmological drops of anesthetic are applied in both eyes and a few minutes before performing the test. The Schirmer test under topical anesthesia measures basal tear flow, eliminating any neural reflex component originating from the surface of the eye. However, to more naturally measure basal tear secretion, the Schirmer test without anesthesia, which measures the basal tear secretion rate with its active reflex tonic component is preferred. However, being unable to quantify the irritative reflex component, the Schirmer test without anesthesia does not accurately measure the contribution of nervous excitation of the nerves on the surface of the eye to tear secretion, or the maximum value that such stimulation can evoke.

Consequently, there is currently no method that allows an efficient evaluation of the maximum secretory capacity of the lacrimal glands in healthy eyes and in diseased eyes, in response to a controlled neural stimulation, that allows defining whether basal tear insufficiency in the surface of the eye, is due to a pathological reduced secretion capacity by the lacrimal glands or to other processes (increased evaporation due to disorders in the composition of the tear, lipid film, or other pathological processes of the ocular surface). Similarly, in patients undergoing surgery of the anterior segment of the eye (photorefractive surgery, cataracts, glaucoma, etc.), the nerves of the ocular surface are inevitably injured to a variable degree, which reduces the ability of these nerves to stimulate basal tear secretion, leading to DED and surface disorders of the eye. Knowledge, prior to surgery, of the maximum secretory capacity of the lacrimal glands of these patients is important to prevent the potential risk of this surgery of generating symptoms of eye dryness. Something similar occurs with contact lens wearers, in which knowing if they have sufficient tear secretion capacity is necessary to avoid the appearance of corneal lesions (see TFOS DEWS II^{®} Report, http://www.tfosdewsreport.org/).

Therefore, knowing the maximum secretory capacity of the lacrimal glands and the degree of reduction of tear flow caused by their inadequate function is important for the diagnosis and prevention of diseases of the ocular surface. The most direct way of knowing this maximum secretory capacity is through supra-maximal stimulation of the activity of the sensory nerves of the ocular surface, which reflexively activate the secretion of these glands.

The most traditional qualitative method used to determine the flow of reflex tear secretion is nasal stimulation using a cotton swab. The device (8 cm long and 3.5 mm thick at the top) is inserted into the nasal cavity, placing it slightly upwards and parallel to the lateral wall. The Schirmer test strip is then placed on the lower eyelid outer third for 5 minutes while the swab is kept in the nasal cavity. A value less than 10 mm is considered to indicate a poor reflex secretion rate. There is also a nasal stimulation device that seeks to reflexively activate the basal tear secretion rate, configured by one or two rods provided at their end with an electrode to produce the discharge of an electrical discharge within the nasal cavity to cause lacrimation. However, in practice this device has a series of adverse effects such as damage to the nasal cavity secondary to the electrical stimulus, nasal bleeding and periorbital discomfort, among others. Thus, both systems of nasal stimulation produce indirect stimulation of uncontrolled intensity and have possible irritative effects on non-ocular tissues.

The ocular pneumo-esthesiometers used to evaluate the sensitivity of the cornea do not establish direct contact with the ocular surface, but rather use a gas flow to stimulate the ocular nerves at a distance (Acosta MC, Tan ME, Belmonte C, Gallar J. Sensations evoked by selective mechanical, chemical, and thermal stimulation of the conjunctiva and cornea. Invest Ophthalmol Vis Sci. 2001; 42, 2063-7; Murphy PJ, Patel S, Marshall JA new non-contact corneal aesthesiometer (NCCA) Ophthalmic Physiol Opt 1996 16: 101-7). By way of example, patent document WO 9412104 describes a procedure that comprises applying to the cornea or conjunctiva of the eye, the sensitivity of which is to be determined, a gas stream containing a mixture of CO₂ and air in variable concentrations to perform a quantitative esthesiometry, serving to determine the threshold to mechanical, thermal and chemical stimuli and the intensity of the discomfort sensations evoked by the stimulus, through verbal responses or the use of a logical scale. This patent also describes equipment for the implementation of the procedure, which provides a continuous flow of the air and CO₂ mixture, which through a three-way valve derives the flow towards an ejector cannula focused on the patient's eye. The appropriate flow is achieved by means of a flow regulator, arranged upstream of the three-way valve, which, based on experimental data or by incorporating a pressure transducer into the equipment, estimates the pressure exerted by the gas mixture (Belmonte C, Acosta MC, Schmelz M, Gallar J. Measurement of corneal sensitivity to mechanical and chemical stimulation with a CO2 esthesiometer. Invest Ophthalmol Vis Sci. 1999 40: 513-9).

It should be noted that esthesiometers such as the one described are designed to measure the threshold sensitivity of the surface of the eye, by means of a controlled compression on it exerted by the projection of pulses of a mixture of gases, for which their purpose is to determine the degree of sensitivity of the patient's cornea (Acosta MC, Tan ME, Belmonte C, Gallar J.Sensations evoked by selective mechanical, chemical, and thermal stimulation of the conjunctiva and cornea. Invest Ophthalmol Vis Sci. 2001; 42, 2063-7). However, this technology is neither directed nor suitable to produce the controlled, sustained chemical stimulation of polymodal nociceptors of the cornea in order to generate a maximum flow of reflex tear secretion, whose measurement allows the diagnosis and prediction of undesired consequences in pathologies. such as DED, systemic autoimmune diseases, the prediction of risks of undesired post-surgical eye surface dryness or the use of contact lenses.

Document WO-A-2006/082588 discloses a device for ophthalmic administration of active pharmaceutical ingredients. The device comprises and a pneumatic circuit connecting an outlet mouth of said gas source with a gas outlet nozzle of the device, the circuit comprising pressure regulation means and flow rate regulation means suitable for ejecting puffs of the gas through the outlet nozzle at a predetermined pressure and flow rates.

Therefore, it would be desirable to have a particularly suitable and simple solution to generate a maximum increase in the flow of reflex tear secretion, obtained through a selective and controlled stimulation of corneal nociceptors, so that the value of the volume of tear secretion obtained (measured by the Schirmer test technique) allows objectively, reproducibly and optimally quantifying the secretory capacity of the lacrimal glands of healthy and diseased eyes; and therefore, that allows to guarantee a correct diagnosis of ophthalmological diseases and to differentiate in a more reliable way healthy subjects from patients with different degrees of tear secretory capacity, such as patients with subclinical dry eye versus those suffering from the syndrome by Sjögren.

It would also be of interest if the device to produce a maximum reflex tear secretion through the chemical stimulation of polymodal corneal nociceptors, were minimally invasive for the patient while avoiding sensations of irritation and discomfort.

### Explanation of the invention

In order to provide a solution to the problems raised, a procedure is disclosed in claim 1 for the generation of a reflex tear secretion.

The procedure comprises the chemical stimulation of the cornea of the eye of a subject by applying to said cornea a controlled jet of gas mixture with a high CO₂ content, of at least the 99% CO₂ in order to produce a transient decrease in pH of the tear film that covers the cornea due to the immediate formation of carbonic acid resulting of the interaction in the tear of the CO₂ with the H₂O of the tear, which leads to the release of protons H + that stimulate the nerve endings of the polymodal nociceptors of the corneal surface, this chemical stimulation giving rise to a maximal reflex tear secretion, whose magnitude is capable of being measured by conventional techniques for the diagnosis of ophthalmological diseases, especially for dry eye disease (DED).

Therefore, maximum stimulation of the nerve endings of the polymodal nociceptors of the corneal surface is achieved through the controlled application of gas puffs with a high CO₂ content, which provides a significant increase in reflex tear secretion flow, compared to baseline tear secretion measured with the Schirmer test as used in the state of the art. Likewise, the rich CO₂ gas jet, upon contact with the corneal surface, can stimulate the mechanoreceptor endings and temporarily reduces the temperature of the cornea, which also stimulates the cold thermoreceptor endings of the cornea, achieving overall a complete stimulation of the sensory nerves modulating tear secretion (Acosta MC, Peral A, Luna C, Pintor J, Belmonte C, Gallar J.Tear secretion induced by selective stimulation of corneal and conjunctival sensory nerve fibers. Invest Ophthalmol Vis Sci. 2004; 45:2333-2336).

Consequently, the determination of the reflex lacrimal secretion flow generated by the procedure of the invention, allows to clearly differentiate the healthy patient from the one affected by DED, even in doubtful cases with a subclinical level of this disease, allowing a better diagnosis and more effective treatment, as well as the identification of patients at risk in whom the application of potentially aggressive therapeutic procedures on the ocular surface (photorefractive surgery, fitting of contact lenses) can lead to the appearance of symptoms of DED.

Likewise, the application of the rich CO₂ jet of gas, being limited to the cornea and being a puff of a very short duration, only evokes at most, a shortlasting sensation of mild discomfort that is very well tolerated by the patient.

Preferably, the outlet pressure of the puff of gas rich CO₂ gas applied towards the corneal surface is higher than the atmospheric pressure. As explained later, the force measured at the output of an outlet nozzle ejecting the gas can be of approximately 6 mN.

Preferably, the jet of gas is applied towards the corneal surface with a flow rate equal to or greater than 150 and more preferably about 200 ml / minute.

Also preferably, the jet of gas is a puff of a short duration applied perpendicularly to the corneal surface for a preset time between 2 and 6 seconds, preferably 4 seconds.

The jet or puff of gas is applied to the corneal surface maintaining a separation distance equal to or greater than 1mm, preferably 10mm.

According to another aspect, the invention also relates to a compact device for the generation of a reflex tear secretion, characterized in that it comprises a gas source in the form of a disposable-type cartridge containing a pressurized gas with a composition of at least 99% of CO₂, and a pneumatic circuit that connects an outlet mouth of said gas source with a gas outlet nozzle of the device. The circuit comprises means for regulating the pressure and flow rate suitable for ejecting puffs of the gas through the outlet nozzle at a predetermined pressure and flow rates, between 1.0 and 1.5 bar and at least 150 ml / minute, respectively. The gas source and the pneumatic circuit are assembled in a housing that can be grasped with one hand or attachable to the clamping bar of instruments of a common ophthalmic examination table, and the device further comprises a user-operable gas puff trigger and a spacer bracket with a proximal end, coupled to the housing around the outlet nozzle, and a distal end, provided with a peripheral edge ergonomically configured to rest on a subject's target eye socket during device's gas shooting, to provide a separation distance, which can be of 1cm, between the gas outlet nozzle and the corneal surface of the subject.

In an embodiment of the invention, the outlet nozzle of the device has an output orifice of 0.5 to 0.1mm diameter with a preferred value of 0.25mm diameter.

According to another characteristic of the device, the pressure regulation means are tightly connected to the outlet of the gas source and configured to regulate the pressure of the gas flow at the outlet of the gas source to a preset value, preferably between 1.2 and 1.6 bar, calculated to provide a predetermined pressure at the outlet of the nozzle not far from the atmospheric pressure, being between 1.0 and 1.5, and preferably of 1.25 bar.

The aforementioned features of the device as the diameter of the outlet nozzle orifice, gas flow, C02 concentration of the gas jet at the nozzle tip and perpendicular position of the output nozzle orifice, at a fixed distance from the center of the cornea determined by the dimensions and shape of the spacer bracket, produces an inverted conical gas jet, whose base covers the surface of the cornea with a CO₂ concentration, pressure and temperature of the gas jet required to obtain a stimulation of corneal nerve terminals sufficient to evoke maximal reflex tear secretion.

According to another feature of the device, the pneumatic circuit also comprises electrovalve means arranged downstream of the pressure regulation means, configured to control the passage of the CO₂ flow towards the outlet nozzle, the flow regulation means being arranged downstream of the solenoid valve means and connected to said outlet nozzle.

According to another characteristic of the device, the pneumatic circuit also comprises pressure sensor means configured to measure the pressure at one or more intermediate points along the gas flow passage circuit.

Preferably, a first gas flow pressure sensor measures the inlet pressure at the entrance and is located between the mechanical regulator/pressure reducer outlet and the cutting solenoid valve. A second sensor is responsible for measuring the output pressure and is located behind the solenoid valve and before the exit needle. The solenoid valve has a mechanical limit of 3.1 bar, but electronically the inlet and outlet pressure are limited between 1 bar and 1.7 bar, with a preferable value of 1.5 bar

According to another characteristic of the device, the pneumatic circuit further comprises solid particle filtering means arranged at one or more intermediate points along the gas flow passage circuit.

According to another characteristic, the device comprises electrical supply means. Preferably, said electrical supply means are configured by a rechargeable electric battery by means of an external charger connected to the electrical supply network.

Advantageously, the device also comprises a light source reference to allow the patient to line up the pupil with the nozzle output and / or acoustic indicator means linked to different status and alarm situations of the device, such as the on or off status, activation of the trigger phase, gas pressure status alarm, load level of the means of electrical supply and a timer to signal the moment for retiring the Schirmer strip, among others.

According to another characteristic, the device comprises control means governed by a microprocessor by means of suitable software, configured to process the signals detected by the pressure sensors during an activated trigger phase, and the signal of the state of charge of the power supply, for the purpose of sending an actuation signal to the solenoid valve means to project a controlled puff of CO₂ through the nozzle, that is, with pre-established conditions of outlet pressure and flow for a predetermined time of stimulation applied to the cornea, and a counter of the number of pulses obtained from the gas bottle. The device can make use of personal area communications (PAN), for example via Bluetooth, allowing its connection with an external device able to perform reading/writing actions through an interface/application to obtain information associated to identification of cartridges using a NFC technology.

Advantageously, the spacer bracket is asymmetrical and rotatable around the outlet nozzle so that it can be applied indistinctly on the socket of right or left eye.

Additionally, the peripheral edge of the spacer bracket intended to come into contact with the eye socket is covered by a protective element made of a biocompatible material, such as silicone, with antibacterial and hypoallergenic properties for greater hygiene and comfort.

Preferably, said protective element is provided with removable fastening means, such as an adhesive, on the face in contact with the spacer bracket to facilitate its placement and removal. Said protective element is disposable and single-use for each subject.

According to a first preferred embodiment, the device is shaped in dimensions such as to make it portable and graspable with one hand, in the form of a pistol or the like, in which the housing is provided with a handle-like portion and a body at whose front end is the outlet nozzle of the CO₂ puffs, and the trigger being integrated in an area of the housing.

In this way, a portable handheld device is obtained, autonomous, ergonomic, light, easy to use, and easy to maintain.

Preferably, the trigger is made up of a trigger positioned at an ergonomic distance from the grip capable of being actuated by at least one finger of the same hand that holds the grip.

Additionally, the housing comprises a removable portion located in the handle, provided with an internal cavity for the placement of a CO₂ cartridge.

According to a second preferred embodiment, the device is shaped to be coupled to the instrument holding bar of a common ophthalmic examination table, such as a slit lamp provided with a frontal support element and a chin rest. In this way, this fixed device makes it possible to apply the CO₂ puff on the eye of a subject when his/her head is held in a fixed position thanks to the frontal support and the chin guard available on such tables.

Likewise, the trigger of this fixed device comprises remote control means separate from the casing, such as an infrared remote control or an actuation pedal, in order to avoid any unwanted movement on the casing when the user presses the trigger to generate the CO₂ puff.

On the other hand, it should be noted that the conventional filter paper strips used in the Schirmer test have a length of 30 mm indicated on a graduated scale printed on their surface. However, the inventors of the present patent application have found that, in some cases, said strips of conventional paper are too short when the reflex secretion with CO₂ is evoked by the device of the invention, since the area moistened by the tear reaches the distal end of the strip.

For this reason, the invention also provides for a set of improved measuring strips for carrying out the Schirmer test, intended for the diagnosis of ophthalmological diseases, especially dry eye disease, said measuring strip being especially suitable for use used to measure the magnitude of maximum reflex tear secretion generated by the device of the invention. Said measuring strip is characterized by being configured by a millimeter filter paper strip with a length greater than 30 mm, the proximal end of which, intended to be inserted into the space between the bulbar conjunctiva and the eyelid, is shaped like a tab with edges rounded to eliminate sharp angles and thus reduce mechanical irritation. Also, the filter paper strip is sterilized, and the end of the tab contains a dried solution of an ocular anesthetic that when wetted by the tear film acts locally, attenuating the foreign-body sensation evoked by the direct contact of the tab with an small area of the lid and eyeball mucosae, but without significant diffusion of the anesthetic to the nerve endings of the eye surface during the time of stimulation.

This set may be marketed together with the device, as a kit, or it may be offered separately as it is a consumable that is especially suitable for use in combination with the device of the invention.

According to a preferred embodiment, the filter paper strip has a width of approximately between 5 and 10 mm and a total length of approximately 50 mm. In this way, a strip of paper of greater length and less width is obtained than the existing strips on the market for the measurement of basal tear secretion, which avoids its saturation when there is a very abundant lacrimation.

### Brief description of the drawings

The attached drawings illustrate, by way of non-limiting example, two preferred embodiments of the device for producing reflex tear secretion, object of the invention. In these drawings:
- Fig. 1 is a perspective view of a portable handheld compact device, according to a first preferred embodiment of the invention;
- Fig. 2 is a side elevation view of the device of Fig. 1;
- Fig. 3 is a front elevation view of the device of Fig. 1;
- Fig. 4 is a top plan view of the device of Fig. 1;
- Fig. 5 is a longitudinal section view of the device of Fig. 1, showing the internal components;
- Fig. 6 is an enlarged view of Fig. 5, showing the connection of the tubes of the CO₂ flow circuit;
- Fig. 7 is a block diagram of the main components of the device according to the first embodiment of the invention;
- Figs. 8 and 9 are two perspective views of a device of essentially horizontal configuration, according to a second preferred embodiment of the invention, attachable to the instrument holding bar on a conventional ophthalmic examination table;
- Fig. 10 is a schematic view of a Schirmer test strip especially suitable for measuring the amount of tear flow secreted by the device of the present invention;
- Fig. 11 is a graph showing the tear secretion values before and after stimulation with CO₂ in the group of healthy subjects;
- Fig. 12 is a graph showing the differences in CO₂ stimulated tear secretion values exhibited by normal and strong responders to CO₂ in the group of healthy subjects;
- Fig. 13 differences in STR within the population of healthy subjects
- Fig. 14 is a graph showing the effect of CO₂ stimulation with the CO₂ stimulation device in subclinical patients;
- Fig. 15 shows the response curve to CO₂ stimulation in the group of Sjögren syndrome patients; and
- Fig.16 compares the mean values of BTR (black) and STR (gray) in the groups of healthy subjects, subclinical DED patients and SS patients.

### Detailed explanation of the invention

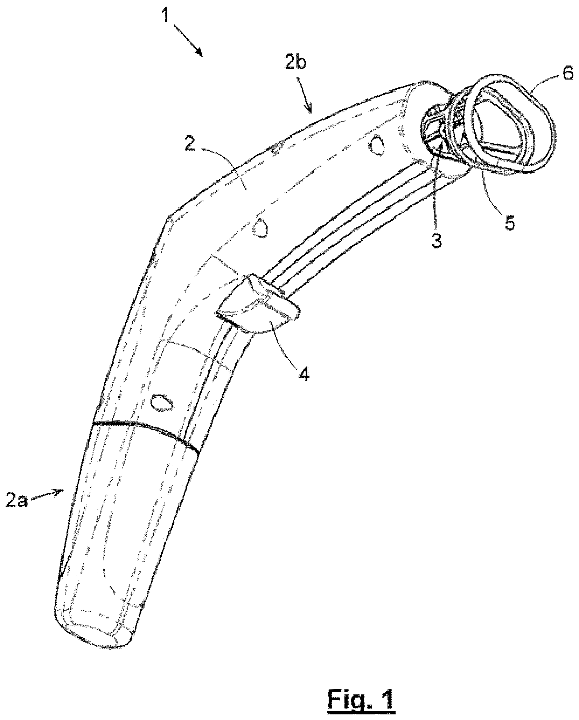

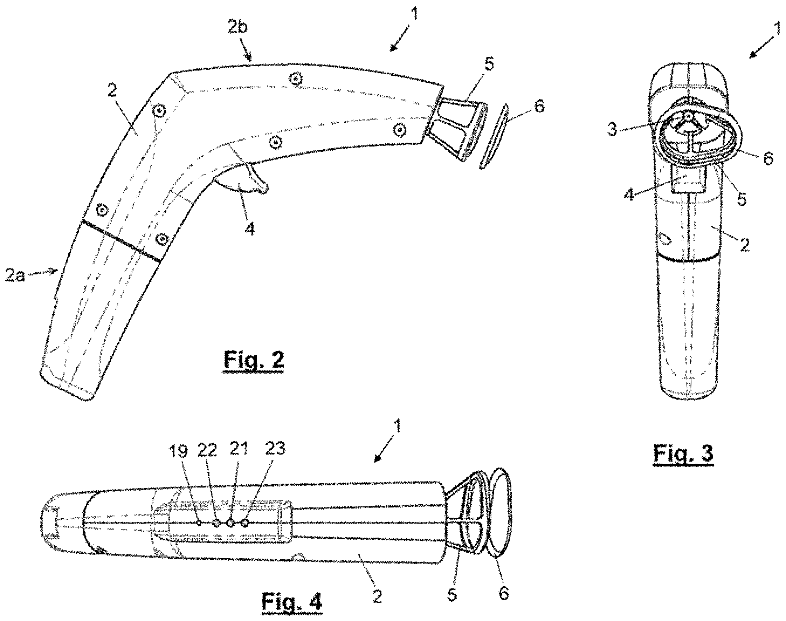

A first preferred embodiment of a device according of the invention for the generation of a reflex tear secretion by means of a chemical stimulation of the corneal surface, is shown in Figures 1 to 6 which illustrate a portable handheld device 1 configured as a pistol.

The device 1 comprises a gas source 7 in the form of a disposable type cartridge containing a pressurized gas with a composition of at least 99% CO₂; and a pneumatic circuit that connects an outlet mouth of said gas source 7 with a gas outlet nozzle 3 of the device 1. The circuit comprises pressure regulating means 8 and flow rate 17 regulation means suitable for ejecting gas puffs through the nozzle outlet 3 at a predetermined pressure and flow rates. The gas source 7 and the pneumatic circuit are assembled in a casing 2. The device 1 further comprises a trigger 4 of the CO₂ puff operable by a user.

In this first embodiment, the device 1 is shaped in dimensions such as to make it portable and graspable with one hand, in the form of a pistol, in which the housing 2 is provided with a handle-like portion 2a and a main body 2b at the front end of which is the outlet nozzle 3 for the CO₂ puffs. Furthermore, the trigger is formed in this example by a trigger 4, which is positioned at an ergonomic distance from the handle 2a in order to be easily actuated by at least one finger of the same hand that holds the handle 2a.

The pressure regulating means 8 are tightly connected to the outlet mouth of the gas source 7 and configured to regulate the pressure of the CO₂ flow at the outlet of the gas source 7 to a pre-established set point, preferably equal to or greater than 1.2 bar, calculated to provide a predetermined pressure at the outlet of the nozzle 3 of approximately atmospheric pressure.

The amount of CO₂ diffused into the air is determined by the CO₂ flow rate and the opening time of the gas outlet in each test. The CO₂ flow rate is regulated to a fixed value and cannot be modified by the user. The opening time of the CO₂ outlet is preset in the device programming and cannot be modified by the user. Depending on the selection of components and the regulation of the device, the CO₂ flow at the outlet of nozzle 3 will be adjustable (at the factory) to a value greater than 50 ml / minute, under ambient conditions (1 bar pressure and 25°C temperature). In this application example, a flow rate of 200 ml / minute applied for 4 seconds is expected.

The CO₂ concentration on the surface of the eye is determined by the distance between the point of diffusion of the CO₂ puff through the outlet nozzle 3 and the surface of the eye, as well as by the degree of dilution of CO₂ in the atmospheric air during its trajectory to the eye.

For this, the device 1 comprises a spacer bracket 5 with a proximal end coupled to the housing 2 around the outlet nozzle 3 and a distal end provided with a peripheral edge ergonomically configured to be supported on the socket of a target eye of a subject during the activation of the device 1 to provide an optimum separation distance between the gas outlet nozzle 3 and the corneal surface of the subject. In this example, the separation distance is 10 mm, although it could vary depending on the set flow and pressure parameters of the CO₂ puff at the outlet nozzle 3. The minimum distance is 1 mm as long as it is used properly, in conjunction with suitable positioning systems.

According to this example, said spacer bracket 5 is asymmetrical and rotatable around the outlet nozzle 3 to be able to carry out the test indistinctly in one eye or the other of the patient. The peripheral edge intended to come into contact with the eye socket is covered by a protective element 6 to improve comfort, and which is easily replaceable for its replacement for each new patient. In this example, the protective element 6 is made of a non-woven fabric made of biocompatible silicone with antibacterial and hypoallergenic properties for greater hygiene and comfort. In addition, it includes a removable adhesive on the face in contact with the spacer bracket 5 to facilitate its removal, being disposable and for single use for each patient.

In the example, a spacer bracket 5 with open walls is shown to allow inspection of the eye during the test. However, a spacer bracket made of a closed-walled body in a transparent material could be used to avoid dispersion of CO₂ while allowing visualization of the eye.

Therefore, the device 1 of the invention is configured to provide a controlled pulse or breath of a gas, with a high content of CO₂, which ensures the stimulation of all polymodal nociceptor corneal nerves, starting from 99% CO₂, with a constant flow rate for a preset time, and maintaining a preset safety distance from the corneal surface, all with the purpose of creating a decrease in pH in the tear film that covers the cornea due to the formation immediate release of carbonic acid due to the interaction of CO₂ with H₂O in the tear, which causes the formation of H + protons that stimulate polymodal nociceptor nerve endings on the corneal surface. This stimulation is perceived in the brain of the patient as a moderate sensation of irritation while activating in the midbrain the nerve centers that mediate reflex tear secretion, the volume of which can be measured with conventional techniques used in the diagnosis of eye diseases specially for dry eye disease.

In this way, the user will perform a test on each patient that consists of applying at least one shot of a CO₂ puff to one or both eyes.

It should be noted that the device of the invention does not provide the user with any type of test result. The result of the test will be evaluated by the user based on the change in the flow of tear secretion experienced by the patient and preferably measured with a Schirmer strip especially suitable to be used in combination with the device of the invention, as will be explained further ahead. Therefore, the device 1 does not perform test evaluation or diagnosis.

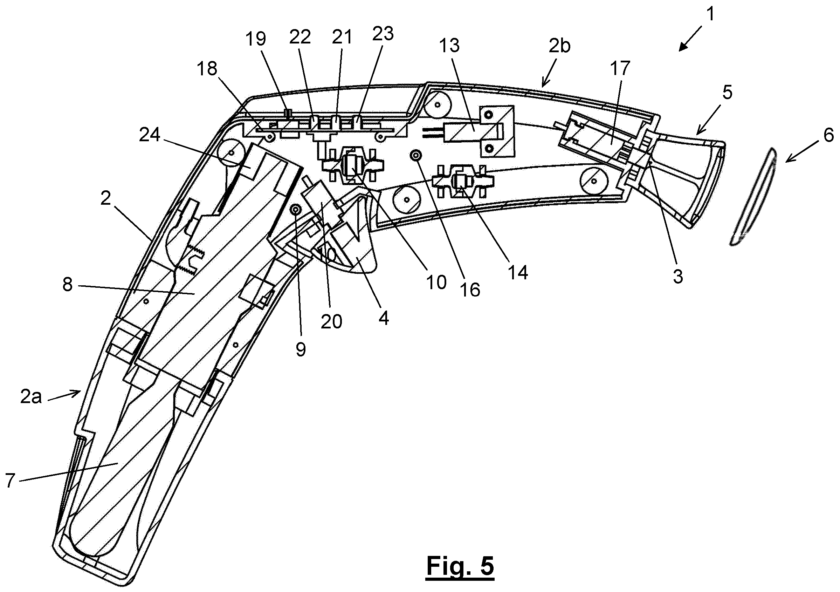

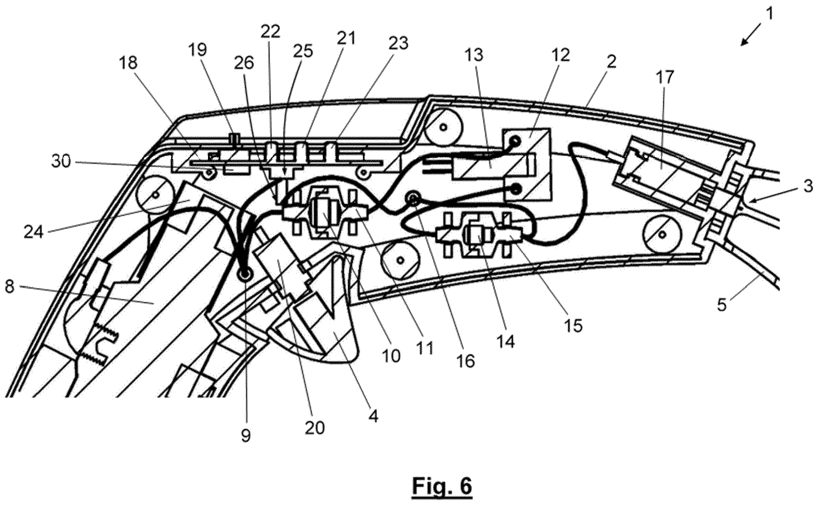

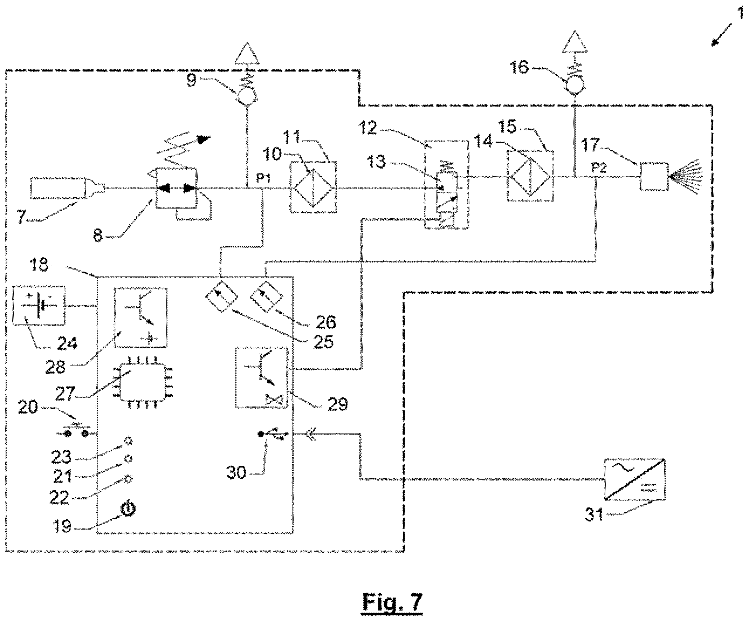

Next, the electronic components of the device 1 are described, referring to Figures 5 and 6, and especially to Figure 7 which shows the block diagram of the main components thereof.

The device 1, according to the first preferred embodiment of the invention, comprises the following components:
- Gas source 7:
   The gas supply source is a disposable CO₂ cartridge 7 (non-refillable). Each CO₂ cartridge can be used for more than one test, even with more than one patient. In this example, a medical grade CO₂-containing steel cartridge is used that contains (at full load) approximately 12 g of CO₂ stored at a pressure of approximately 50 bar. The cartridge is provided of an identification system that prevents the use of the device with unauthorized cartridges.
- Pressure regulation means 8.
   In this example, a gas pressure regulator is used that has an inlet port for a 3/8" threaded CO₂ cartridge. It allows regulating the gas outlet pressure of cartridge 7 to the working pressure (set pressure) of the device 1 subsequently blocking the regulation setting. This regulation of the working pressure will be carried out in manufacturing and should not be manipulated by the user.
- Inlet safety valve 9.
   Mechanical pressure relief valve. Protects the circuit from accidental pressures higher than the pressure set on the pressure regulator. The CO₂ is released to the outside through an opening in the housing 2 of the device 1. The valve 9 is regulated by means of a spring set at the adequate pressure. Once the pressure returns to its normal value, valve 9 closes automatically.
- Inlet filter 10.
   Metallic screen (AISI 316) to prevent solid particles from entering the circuit that could cause damage to a solenoid valve 13 arranged downstream, or to the patient. The screen pitch is 40 microns.
- Inlet filter housing 11.
   Housing for inlet filter 10. It has inlet and outlet connections for CO₂.
- Manifold 12.
   It supports a solenoid valve 13 and allows connecting its inlet and outlet ports.
- Solenoid valve 13.
   A solenoid valve 13 is used to control the opening of the CO₂ diffusion circuit in the patient's eye. It is a 2-way, 2-position solenoid valve of the bistable type. It incorporates a 12-aluminum manifold. The actuation of the solenoid valve 13 is controlled by 2 signals generated by a microprocessor 27 and subsequently driven by a solenoid valve driver 29, both arranged on an electronic control board 18, which will be described later.
- Outlet filter 14.
   Metal screen (AISI 316) to prevent solid particles from entering the circuit that could cause obstruction in a flow rate 17 regulation means arranged downstream or reaching the patient. The screen pitch is 40 microns.
- Outlet filter housing 15.
   Housing for outlet filter 14. It has CO₂ inlet and outlet connections.
- Outlet safety valve 16.
   Mechanical pressure relief valve. Protects the circuit from accidental pressures higher than the set pressure set on the pressure regulation means 8, here a pressure regulator. The CO₂ is released to the outside through an opening in the housing 2 of the device 1. The valve 16 is regulated by means of a spring set at pressure adequate. Once the pressure returns to its normal value, valve 16 closes automatically.
- Flow rate 17 regulation means
   In this example, an outlet flow restrictor is used, which comprises the nozzle 3 or calibrated CO₂ dispensing needle (in diameter and length) that allows, for a given pressure, to maintain the CO₂ outlet flow at a value predefined.
- Electronic control board 18.
   Electronic board 18 that incorporates all the necessary components to control the device 1, basically:
   - On / off push button 19 of device 1
   - Li-lon battery 24 and its charging circuit 28
   - Microprocessor 27
   - Inputs: digital trigger button 20, analog input pressure sensor 25 and output pressure sensor 26.
   - Outputs: Actuation of solenoid valve 13 through solenoid valve driver 29.
   - Light indicators 21, 22 and 23.
- On / off button 19.
   If the device is off, pressing it for a while turns the device on.
   If the device is on, pressing it for a while turns the device off.
   This button also allows you to leave the device in a very low consumption standby or "sleep" state, either by pressing it for a while or after a predefined period of inactivity.
- Trip button 20.
   Pushbutton which, when activated by the user (by means of trigger 4), generates a digital input signal to the electronic control board 18.
   This signal is used by the microprocessor 27 to start the test, if all the necessary conditions are met, and therefore generate the CO₂ puff, as will be explained later.
- Power indicator 21.
   LED type indicator (Green).
   On in continuous mode, indicates that device 1 is on.
   Lit in oscillating mode, indicates low battery charge level 24.
- Battery charge status indicator 22 24.
   LED type indicator (Blue) that allows differentiating the different states of the battery charging process 24: charging and charged.
- Trip / Pressure Indicator 23.
   Two-color LED indicator (Green / Red).
   - In green color, it turns on continuously during the CO₂ shooting period.
   - In green, it lights up in oscillating mode to indicate low pressure in the CO₂ circuit.
   - In red color, it lights in continuous mode to indicate obstruction in the CO₂ circuit.
   - In red color, it lights up in oscillating mode to indicate excessive pressure in the CO₂ circuit.
- Battery 24.
   24 rechargeable lithium-ion battery, with a nominal voltage of 3.7V and an approximate capacity of 165 mA / hour. In this example, the battery is integrated so it is not accessible to the user.
- Inlet pressure sensor 25.
   Differential type pressure sensor 25 and analog output. It measures the pressure P1 at the outlet of the pressure regulation means 8 so that the microprocessor 27 can verify that it is adequate. It is used to detect empty cartridge 7, excessive regulation means 8 outlet pressure or CO₂ circuit clogging.
- Outlet pressure sensor 26.
   Pressure sensor 26 of differential type and analog output. It measures the pressure P2 at the inlet to the CO₂ outlet flow restrictor so that the microprocessor 27 can verify that it is adequate. It is used for the detection of excessive pressure in the flow restrictor or obstruction of the CO₂ circuit.
- Microprocessor 27.
   It processes the status information of the device and, based on the inputs, manages the status of the outputs. It includes the necessary control software program for its operation that will manage the input signals (digital and analog) and the output signals (actuation of the solenoid valve and LED indicators).
- Battery charging circuit 28.
   Monitors the battery charge 24. Monitors the operation of the battery charge status indicator 22.
- Driver 29 of solenoid valve.
   It conditions the opening and closing signals of the solenoid valve 13 (generated in the microprocessor 27) to adapt them to the requirements of the solenoid valve 13.
- USB 30 input for battery charging.
   Micro USB connector, compatible with standard USB chargers. Just use the power pins. Data pins do not connect. Alternatively, another type of connector could be used such as a Jack connector.
- External charger 31.
   Standard charger for devices with Micro USB charging input, or alternatively with charging input for a Jack connector.
   Input voltage: 110/220 V; 50/60 Hz.
   Output voltage: continuous = 5 V.

The handheld device 1 is therefore powered by batteries 24 and is normally used without connection to the electrical network. In case of low battery level, the device 1 can also be used while connected to the mains to recharge batteries 24. Recharging the batteries 24 will be carried out by using the external charger 31 connected to the mains power supply - low voltage (110/220 V; 50/60 Hz).

Likewise, the housing 2 of the device 1 comprises a removable portion, located in the area of the handle 2a, provided with an internal cavity for the placement of a CO₂ cartridge that can be coupled to the pressure regulation means 8 by means of mutual coupling means, such as threading means, which guarantee the sealing of the cartridge in its position of use.

Next, with reference to Figures 6 and 7, the operation of the device 1 of the invention is briefly described.

The CO₂ cartridge 7 supplies the pressure regulation means 8, here a pressure regulator, with a flow of CO₂ at high pressure (between 50 bar when the cartridge is completely full and 0 bar when it is empty). To do this, the cartridge 7 should be screwed into the threaded inlet port of the pressure regulator. This should be done as quickly as possible to avoid excessive gas loss.

The pressure regulator will adjust its outlet pressure according to the set point that has been set in the manufacture of the device 1. This pressure will be greater than or equal to 1.2 bar (17.4 psia).

The output of the pressure regulator is derived by means of a tube in three connections:
- To the inlet safety valve 9 so that, if the inlet pressure exceeds the maximum safety established, the gas would escape directly to the outside, avoiding possible damage to the circuit, the user or the patient.
- To the inlet pressure sensor 25 located on the electronic control board 18, so that the microprocessor 27 can signal possible failures in the gas circuit (low pressure, excessively high pressure or blockage of the circuit) by means of the corresponding indicator 23.
- To the housing 11 of the inlet filter 10 of the CO₂ circuit.
   Once the CO₂ flow passes through the casing 11 of the inlet filter 10, the outlet of the casing 11 of the inlet filter 10 is connected by tube to the inlet of the manifold 12 of the solenoid valve 13.

The outlet of the manifold 12 is connected by tubing to the inlet of the housing 15 of the outlet filter 14.

The outlet of the casing 15 of the outlet filter 14 is derived in three connections by tube:
- To the outlet safety valve 16 so that, if the inlet pressure exceeds the maximum safety established for the outlet, the gas would escape directly to the outside, avoiding possible damage to the circuit, the user or the patient.
- To the outlet pressure sensor 26 located on the electronic control board 18. The objective is to check that the outlet pressure is adequate to achieve the desired CO₂ flow rate and that the microprocessor 27 can signal possible failures in the gas circuit (low pressure, excessively high or blockage of the circuit) by means of the corresponding indicator 23.
- To the outlet flow rate 17 regulation means, here the flow restrictor, for the emission of CO₂ to the outside with the pre-established flow.

In the example described, the device 1 can be in three normal states (standby; on; and shooting), in two warning states (low pressure on, and obstruction on) and in an alarm and blocking state (excessive pressure). Each of these states is briefly described below:
- Standby state: LEDs 21,22,23 off and non-essential peripherals power supply disabled; only the power supply to the microprocessor 27 is active, but it remains in a "sleep" state, with its clock and peripherals deactivated. It is possible to exit of this state (going to the "on" state) by pressing the on / off button 19 for a sufficient time (about 2 seconds).
- On state: System power supply is fully activated and with microprocessor 27 and its internal oscillator running.
- Shooting state: This state is accessed by activating trigger 4 under the appropriate operating conditions (appropriate battery level for correct operation and input pressure P1 between predetermined upper and lower limits of the set point).
- Low pressure on state (warning): This status is accessed when detecting a low inlet pressure problem P1 in the CO₂ circuit.
- Obstruction-on status (warning): This status is accessed when detecting an obstruction problem in the CO₂ circuit (detected during the shooting state).
- Excessive pressure state (alarm and blocking): This status is accessed when detecting an inlet pressure P1 of CO₂ flow higher than the maximum allowed. This problem can be detected at any time while the device is not in sleep state.

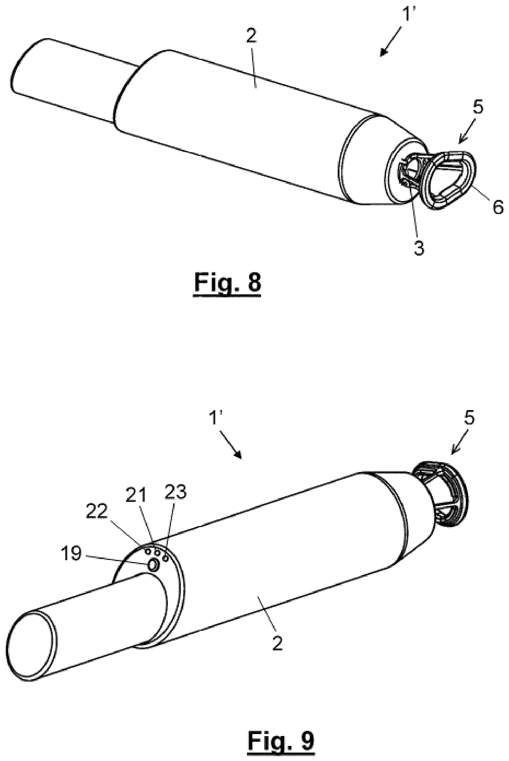

A second preferred embodiment of the invention is shown in Figures 8 and 9 illustrating a fixed device 1' of essentially horizontal configuration, fixedly attachable to the instrument holding bar of a conventional ophthalmic examination table (not shown), as per example a slit lamp. In this way, this fixed device 1' makes it possible to apply the CO₂ puff on the patient's eye when his head is kept in a fixed position thanks to the frontal support element and the chin rest provided by such examination tables. The same reference numerals have been used to identify those elements common to the handheld portable device 1 of the first embodiment.

Likewise, the trigger of this fixed device 1' comprises remote actuation means (not shown) separate from the casing 2, such as an infrared remote control or an actuation pedal, in order to avoid any unwanted movement on the housing 2 when the user presses the trigger to generate the CO₂ puff.

As mentioned above, the conventional filter paper strips used in the Schirmer test have a length of 30 mm indicated on a graduated scale printed on their surface. The inventors of the present patent application have found that, in some cases, said strips of conventional paper are too short when the reflex secretion with CO₂ is evoked by using the device of the invention, since the area moistened by the tear reaches the distal end of the strip.

For this reason, the invention also provides for a set of improved measuring strips 40 for the performance of the Schirmer test, especially suitable for carrying out the measurement of the maximum reflex tear production generated by the device 1,1' of the invention. For this purpose, some strips 40 of absorbent paper are available, millimeter-sized of greater length and less width than those existing in the market for the measurement of basal tear secretion, in order to avoid saturation when there is a very abundant tear.

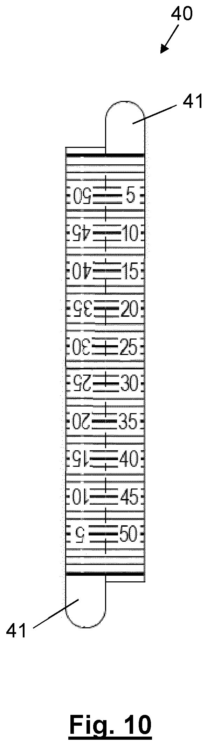

In Fig. 10 an improved strip 40 according to the invention is shown by way of example, which is formed by a strip 40 of filter paper (for example, Whatman^{®} No. 41) with a width of approximately between 5 and 10 mm and with a total length of approximately 50 mm, the proximal end of which intended to enter the space between the bulbar conjunctiva and the eyelid has a tongue-like shape 41 with rounded edges to eliminate sharp angles and thus avoid irritation. Furthermore, said strips 40 are sterilized with ethylene oxide and the end of the tab 41 contains a dried solution of an ocular anesthetic.

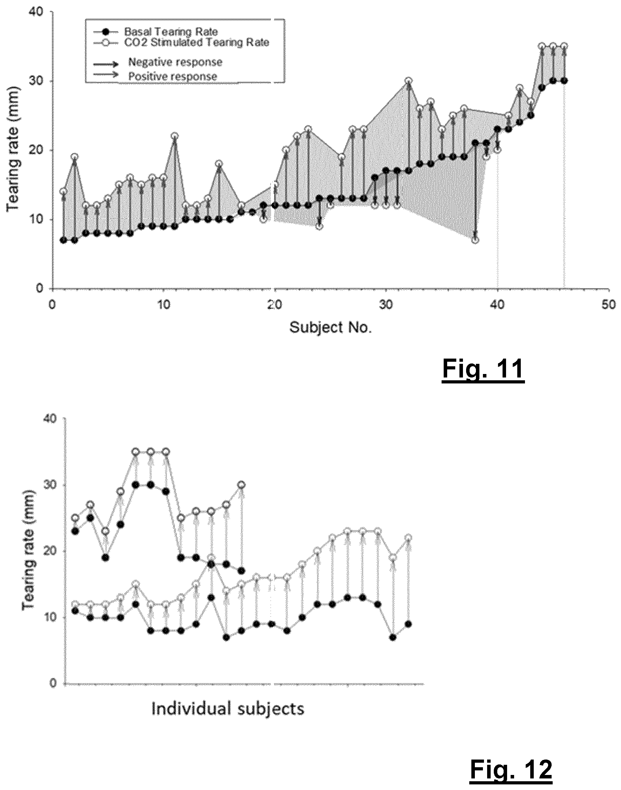

Figure 11 shows the effect of CO₂ stimulation with the device on the tear rate of healthy subjects. Individual values of BTR (black dots) and STR (white dots) of each patient are connected by an arrow and ordered sequentially by their BTR magnitude. Positive and negative values of Tearing reserve (Tr) have been wrapped in red and blue respectively, to emphasize the increased incidence of negative tear reserve values among subjects with higher BTR values.

Figure 12. Healthy subjects ordered according to the magnitude of their positive tearing reserve (Tr). Subjects with BTR < 18 mm (red arrows) or ≥ 18 mm (green arrows) have been plotted apart. Subjects with zero or negative Tr values have been excluded.

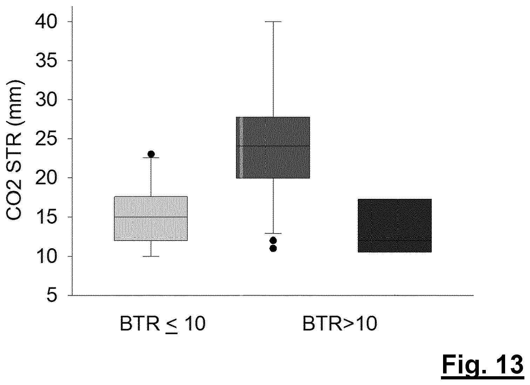

Figure 13 shows the differences in STR within the population of healthy subjects. Healthy subjects with a basal tear Schirmer value below or equal to 10 mm maintain a positive response to maximal reflex stimulation (light red box). Healthy subjects with BTR values over 10 mm segregate in two distinct groups, those in which stimulation does not augment or decrease tearing (blue box) or keep a large tearing reserve (red box). STR values of subjects with BTR>10mm (+) were different from those with BTR<10 mm (Kruskal Wallis, Dunn's method, p<0.05).

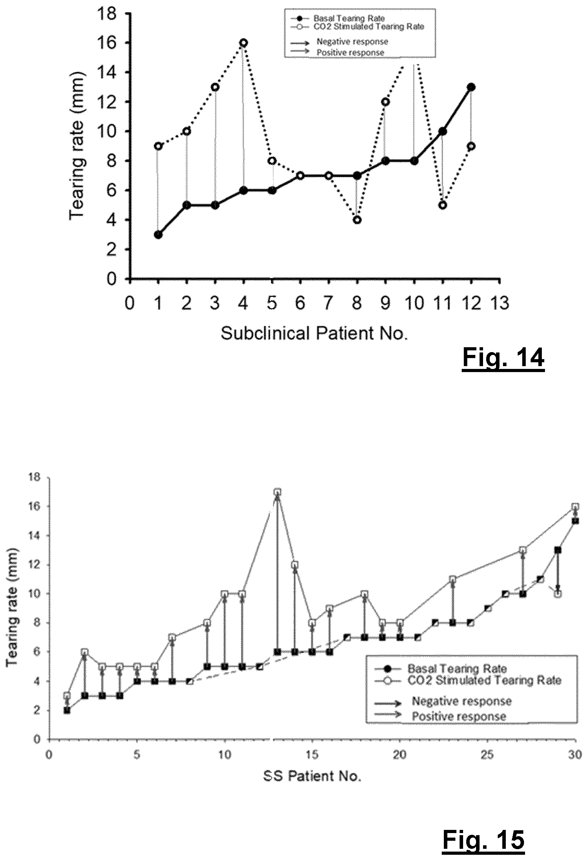

Figure 14 shows the effect of CO₂ stimulation with the CO₂ stimulation device in subclinical patients. As in Figure 11, Individual values of BTR (black circles) and STR (white circles) of each patient are connected by an arrow, and ordered sequentially by BTR magnitude. Positive and negative values of tearing reserve have been wrapped in red and blue respectively.

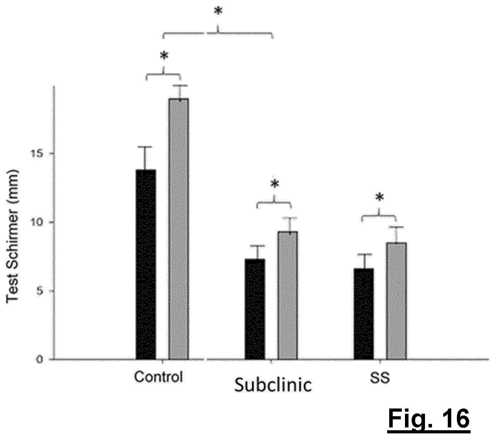

Figure 15 shows the response curve to CO₂ stimulation in the group of Sjögren syndrome patients. Data have been represented as in Figure 11. Individual values of BTR (black squares) and STR (white squares) of each patient are connected by an arrow and ordered sequentially by BTR magnitude. Positive and negative values of tearing reserve have been wrapped in red and blue respectively. Figure 16 is a comparison of the mean values of BTR (black) and STR (gray) in the groups of healthy subjects, subclinical DED patients and SS patients *p<0.05 Mann Whitney test.

### Examples

The inventors evaluated the efficacy and reproducibility of the device to stimulate tear secretion, measuring in human subjects of both sexes the tear secretion volume evoked by maximal reflex stimulation of the lacrimal glands obtained by stimulation of corneal nociceptors and cold thermoreceptors of the ocular surface with the device described in the present invention in subjects of both sexes, healthy and with eye dryness at two levels of severity: Subclinical and with Sjögren's Syndrome (SS) diagnosed using basal Schirmer's test values, tear meniscus size; tear film breakdown time (TBUT), corneal staining level graded with the Oxford scale and Mcmonnies symptomatology questionnaire. Two groups of patients were used:
Group 1 (control), comprised healthy and asymptomatic volunteers of both sexes, aged between 20 and 69 years (n=53) with Schirmer test> 8 mm in 3 min., TBUT> 7 s. and 0 degrees of superficial punctate keratitis (SPK).
Group 2 (ophthalmological patients) included 46 subjects of both sexes, aged between 20 and 69 years, who in turn were divided into:
   A. Subclinical DED patients, with Schirmer test <10 mm in 3 min and the TBUT <7 s (n=13).
   B. Sjögren's Syndrome (SS) patients, diagnosed of dry eye associated with primary or secondary SS, according to the standard criteria established for the identification of this pathology (n=30).

Schirmer test values were obtained under basal conditions (basal tear rate, BTR) in one eye and after 10 min in the contralateral eye immediately after application of a 4s CO₂ pulse with the device of the present invention (stimulated tear rate STR), in control subjects and in patients with subclinical asymptomatic low BTR and with severe dry eye disease (SS).

Immediately before applying the Schirmer paper strip, 3µl of 2% lidocaine was instilled with a micropipette to the flap the strip in contact with the conjunctiva in order to locally anesthetize only this area palpebral mucosa, thus avoiding mechanical irritation of the conjunctiva. BTR was measured for 3 min under resting conditions.

The reflex tear secretion evoked by CO₂ stimulation was performed 10 min later in the eye contralateral to that of the control measurement. For this, the eye socket was applied on the orbit and the patient was asked to look directly to the outflow orifice. Then a 99% CO₂ pulse was applied. Immediately afterwards, a Schirmer strip with its tab impregnated with 3µl of 2% lidocaine was placed on the lower lid of the stimulated eye, maintaining it for 3 min and to obtain the STR. Noteworthy, in the usual practice of the Schirmer test under anesthesia, 10 µl of 0.4% oxybuprocaine is instilled in the conjunctival sac and 5 minutes are waited before performing the Schirmer test.

CO₂ stimulation evoked a significant tear flow increase. The average STR of the positive responders was 20.8±1.3 mm, which represents a 46.5% increase of their mean BTR value (14.2±1.1 mm (p<0.001, Wilcoxon test). No statistical differences of BTR and STR values were found between male and female healthy subjects (p=0.981 and p=0.306 respectively, Mann Whitney test).

In Figure 12, the individual values of BTR and STR of all members of healthy subjects' group (n=46), have been ordered according to their BTR value. This representation evidences the high incidence (72.4% of the total) of positive responses (red arrows) to the stimulus and also that the number of subjects in which STR values evoked by CO₂ do not overpass their BTR was low. The amplitude of the STR value was rather variable, ranging from 5 mm to a highest peak value near 40 mm. Figure 12 also shows that null or negative responsiveness to the stimulus was not observed in any of the healthy subjects whose pre-stimulus BTR value was below 10 mm, but tend to appear with increasing frequency and with larger negative values among subjects with higher pre-stimulus BTR level.

In each healthy subject, we measured the difference between BTR and STR, a parameter that we named Tearing reserve (Tr) and is usually positive in healthy subjects (red arrows, Figure 12). All subjects with low pre-stimulus BTR values, between 7 and 10 mm (n = 16) responded positively to the stimulus, with a mean Tr of +6.8 ± 0.9 mm. Among subjects showing BTR levels over 10 mm (n = 30), two-thirds were still able to augment tearing with CO₂ stimulation, with a similar mean Tr= +7.1 ± 0.8 mm while the rest exhibited a negative Tr (blue arrows, Figure 12). Noteworthy, within the group of fourteen subjects showing a BTR ≥18mm, eleven still produced vigorous tearing in response to CO₂ stimulation, reaching a mean Tr value of +6.6 ± 1.0 mm (n=11) (Figure 13, green arrows). Figure 13 highlights the remarkable capacity of this subgroup of subjects of producing large total tear volumes. There, BTR and STR values of positively responding healthy subjects have been ordered according to the magnitude of their Tr value but plotting separately subjects with BTR value below 18 mm (red) or over 18 mm (green). The graph shows that a subgroup of subjects exhibiting a high BTR still maintain the capacity to respond with a marked increase of their tear secretion (i.e. of keeping a normal tearing reserve value) thus being able to evoke higher final STR values in comparison with subjects with a lower BTR. Accordingly, the mean value of STR (30.0 ± 2.0mm) in this subgroup of strongly responding subjects with an already high BTR (≥18mm) is significantly higher than in subjects responding positively to the CO₂ stimulation with a BTR <18mm (17.0 ± 0.9 mm; p<0.001, Mann Whitney test)

In Figure 14 we represented the mean STR of all healthy subjects. Those with a BTR ≤ 10 mm (34.8% of the total, light red box), responded positively to CO₂ stimulation. Subjects with higher BTR values could be subdivided between those unable to further increase tearing or even exhibiting a lower tearing rate when stimulated (17.4% of the total, blue box), and the group maintaining the capacity to increase tearing under stimulation (47.8% of the total, red box).

Altogether, our data indicate that individual differences in total tear secretion capacity in healthy subjects are ample and that subjects considered normal by their BTR value may actually be close to their maximal tearing capacity.

The group of individuals subclinical DEP patient was characterized by a BTR < 7mm and/or a TBUT < 7s, low tear meniscus height and a moderate level of corneo-conjunctival epitheliopathy clinically suggestive of moderate DED, but without subjective signs of the disease according to their scores in the McMonnies questionnaire.

The change of tearing rate evoked by CO₂ stimulation in 12 patients belonging to this non-symptomatic group is shown in Figure 14. The profile of their response curve is qualitatively very similar to the one obtained in healthy subjects (compare with Figure 11). However, in contrast with them, in subclinical DED patients, the incidence of negative responses or no response to CO₂ stimulation was higher, already appearing in subjects with low BTR values. Moreover, only in two of the subclinical patients the value of peak STR overpasses 16 mm. Still, low tear secretion under basal conditions in the patients with BTR below 7 mm, apparently does not totally compromise the capacity of their lacrimal glands to increase secretion under nociceptive stimulation. In fact, the average tearing reserve (Tr) value of the group was +6.1 ± 1 mm, n = 7, only slightly lower than the equivalent Tr value in healthy subjects with a BTR below 10 mm (compare Figures 11 and 14). However, more than half of the subclinical DED patients were unable to reach a peak tear volume of 10 mm under CO₂ stimulation. Statistical analysis showed no significant correlation between BTR and STR in subclinical DED subjects (p=0.6, Pearson correlation coefficient = -0.168), but showed a negative correlation between BTR and Tr (p=0.024, Pearson correlation coefficient = - 0.642).

The group of SS-DED patients was composed of 30 patients derived to Ophthalmology by the Service of Rheumatology and diagnosed as primary SS (9 patients) or secondary SS (21 patients).

The individual responses to CO₂ of all the members of the SS group are shown in Figure 15. There, patients have been ordered as a function of their BTR value as in control and subclinical groups. Up to BTR values ≤ 7 mm, most patients (17/21) exhibited a positive albeit modest tearing response to CO₂ stimulation (mean rise: +3.1 ± 0.6 mm, n = 17). For BTR >7 mm only three out of 9 patients increased their Schirmer test score in 1-3 mm, while the rest remained unchanged or decreased. Taken together, these data indicate that the Tr of SS patients is in general very low, and that the BTR appears to be at or near maximal level already under basal conditions in about 50% of the patients.

Figure 16 summarizes the mean values of BTR and STR obtained in healthy, subclinical DED subjects and SS-DED patients, confirming that SS-DED patients exhibit significantly lower tear secretion under basal conditions, but also showing that in these patients, despite the functional insufficiency of their tear glands, a capacity to increase tear flow under maximal reflex stimulation still remains. A similar secretion pattern is also observed in subclinical patients, in which slightly higher values of BTR and STR apparently prevent the apparition of open DED symptoms.

We report here the results obtained with a new ophthalmic instrument that generates a CO₂-evoked maximal stimulation of the sensory nerve terminals innervating the corneal surface, thereby evoking a strong reflex activation of tear gland's secretion and a rise in the aqueous tear flow. This sudden increase of the tear volume possibly represents the maximal acute secretory capacity of the gland. Comparison of the results obtained with this procedure between healthy subjects and patients diagnosed of DED, evidence that the elevation of tear flow over its basal levels evoked by CO₂ stimulation was very low in Sjögren syndrome patients displaying defined DED ocular signs and symptoms, but also in patients showing moderate signs of DED such as abnormal tear film stability and volume, and discrete corneal epithelium damage, but without complaining of subjective symptoms of DED. Thus, measurement of maximal reflex tear gland secretion with this simple procedure may provide useful information about the functional status of patient's tear glands and their capacity to prevent the development of ocular surface dryness. It may be useful also to follow and predict an evolution towards DED in subjects with a low BTR but without symptoms of the disease. Almost all of them were unable to increase their basal tearing rate under stimulation, and the STR of those responding positively did never overpass the mean value of BTR exhibited by healthy subjects. The response curve closely resembles the one of the SS patients, thus reinforcing the tenet that these are probably patients in a masked, early stage of lacrimal gland insufficiency. Clinically, SS patients' basal tearing levels are commonly used as an important functional sign of the magnitude of lacrimal gland damage produced by an autoimmune disorder. Our data indicate that in contrast with healthy subjects, most SS patients have a maximal secretory capacity moving in a narrow range away from the BTR and rarely overpassing 12mm. A corollary of this observation is that therapeutic procedures aimed at increasing the tearing reserve of SS patients to enhance their low basal tearing should take into consideration the actual range of potential tear volume increase still available for these patients.

Altogether, our observations support the tenet that maximal tear flow values evoked with reflex chemical stimulation of corneal nerves reflect with reasonable fidelity the functional status of the tear secretory glands. Moreover, values of maximal secretory capacity and tearing reserve may be useful to predict the functional capability of lacrimal glands to respond to environmental challenges and to react against a variety of systemic or eye disorders that are accompanied by ocular surface infection and/or inflammation, wherein insufficient tear flow contributes to aggravate the altered condition of the eye surface. It is well known that reflex compensatory tearing in response to ocular surface stress following surgery or under prolonged contact lens wearing is important to prevent injury of the corneal epithelium and its nerves and the accompanying inflammation and unpleasant dryness and pain sensations. Knowledge of lacrimal gland secretory condition using controlled reflex stimulation of the eye surface in prospective patients of photorefractive surgery or contact lens users may help eye care professionals to adopt informed decisions about the convenience and potential risks of these procedures in some patients before applying them to improve the refractive state of the eye.

## Claims

1. Procedure for measuring the generation of a maximal reflex tear secretion, comprising chemical stimulation of the cornea of the eye of a subject consisting in applying towards said cornea a controlled jet of a gas mixture with a high CO₂ content, of at least the 99% CO₂, in order to produce a transient decrease in pH of the tear film that covers the cornea, due to the immediate formation of carbonic acid, resulting of the interaction in the tear of the CO₂ with the H₂O of the tear, which leads to the release of protons that stimulate the nerve endings of the polymodal nociceptors of the corneal surface, this chemical stimulation, combined with the mechanical and cold stimulation evoked by the gas jet, lead to a maximal reflex tear secretion, whose magnitude can be measured by conventional techniques for tear flow measurement used in Ophthalmology and Optometry for the diagnosis of tear secretion disturbances of ophthalmological diseases, especially for dry eye disease, wherein the jet or puff of gas is applied towards the corneal surface maintaining a separation distance equal to or greater than 1mm.

2. Procedure for measuring the generation of a reflex tear secretion according to claim 1, **characterized in that** the outlet pressure of the jet of gas applied towards the corneal surface is higher than the atmospheric pressure.

3. Procedure for measuring the generation of a reflex tear secretion according to any one of the preceding claims, **characterized in that** the jet of gas is applied towards the corneal surface with a flow rate equal to or greater than 150 ml / minute, preferably 200 ml / minute; and **in that** the jet of gas is a puff of a short duration applied in the direction of the corneal surface for a preset time comprised between 2 and 6 seconds, preferably 4 seconds.

4. Procedure for measuring the generation of a reflex tear secretion according to any one of the preceding claims, **characterized in that** the jet or puff of gas is applied towards the corneal surface maintaining a separation distance equal to or greater than 10mm.

5. Compact device (1,1') for the generation of a reflex tear secretion, comprising a gas source (7) in the form of a disposable-type cartridge containing a pressurized gas with a composition of at least 99% of CO₂; and a pneumatic circuit connecting an outlet mouth of said gas source (7) with a gas outlet nozzle (3) of the device (1,1'), the circuit comprising pressure regulation means (8) and flow rate (17) regulation means suitable for ejecting puffs of the gas through the outlet nozzle (3) at a predetermined pressure and flow rates, between 1 and 1.5 bar and at least 150 ml / minute, respectively, the gas source (7) and the pneumatic circuit being assembled in a casing (2) that can be grasped with one hand or can be attached to the instrument holding bar of a common ophthalmic examination table, and the device (1,1') also comprises a user-operable gas puff trigger (4) and a spacer bracket (5) with a proximal end, coupled to the housing (2) around the outlet nozzle (3), and a distal end, provided with a peripheral edge ergonomically configured to be supported on the target eye socket of a subject while firing the device (1.1'), to provide an separation distance between the gas outlet nozzle (3) and the corneal surface of the subject.

6. Compact device (1,1') according to the claim 5, **characterized in that** the pressure regulation means (8) are tightly connected to the outlet of the gas source (7) and configured to regulate the pressure of the gas flow at the outlet of the gas source (7) to a pre-established set point, between 1.2 and 1.6 bar, to provide a predetermined pressure at the outlet of the nozzle (3) of approximately between 1.0 and 1.5, preferably 1.25 bar.

7. Compact device (1,1') according to any one of claims 5 or 6, **characterized in that** the pneumatic circuit further comprises a first pressure sensor (25) designed to measure the intermediate pressure (P1) existing at the outlet of the pressure regulation means (8); and a second pressure sensor (26) designed to measure the intermediate pressure (P2) existing at the entrance to the flow rate (17) regulation means, and **in that** the pneumatic circuit also comprises safety valve means (9,16) configured to ensure the evacuation of the flow of gas when the pressure (P1, P2) detected by the pressure sensors (25,26) is higher than the set pressure value at the respective intermediate measurement points.

8. Compact device (1,1') according to any one of claims 5 to 7, **characterized in that** it comprises control means governed by a microprocessor (27) provided of a suitable software, configured to process the signals detected by the pressure sensors (25,26), during an activated trigger phase, and the signal of the state of charge of an electrical supply means (24), with the purpose of sending an actuation signal to a solenoid valve (13) arrange downstream of the pressure sensors (25, 26) to project through the nozzle (3) a controlled puff of gas, that is, with pre-established conditions of outlet pressure and flow for a predetermined time applied in the direction of the cornea of a subject.

9. Compact device (1,1') according to the preceding claim, **characterized in that** the microprocessor (27) is equipped with a personal area communications (PAN), allowing its connection with an external device able to perform reading/writing actions through an interface/application to obtain information associated to identification of cartridges using a NFC technology.

10. Compact device (1,1') according to any one of claims 5 to 9, **characterized in that** the spacer bracket (5) is asymmetric and is rotatable around the outlet nozzle (3) to be able to be used interchangeably in one eye or the other of the subject.

11. Compact device (1,1') according to any one of claims 5 to 9, **characterized in that** the peripheral edge of the spacer bracket (5) intended to come into contact with the eye socket is covered by a protective element (6) made of a biocompatible material, such as silicone, with antibacterial and hypoallergenic properties for greater hygiene and comfort.

12. Compact device (1,1') according to any one of claims 5 to 11, **characterized in that** it is shaped in dimensions such as to make it portable and grasp with one hand, in the form of a pistol or similar, in where the casing (2) is provided with a handle-like portion (2a) and a main body (2b) at the front end of which is the outlet nozzle (3) of the CO₂ puffs, and the trigger being (4) integrated in an area of the housing (2).

13. Compact device (1) according to claim 12, **characterized in that** the casing (2) comprises a removable portion located in the area of the handle (2a), provided with an internal cavity for the placement of a CO₂ cartridge (7).

14. Compact device (1') according to any one of claims 5 to 13, **characterized in that** it is shaped to be coupled to the instrument holding bar of a common ophthalmological examination table, and in wherein the trigger comprises remote actuation means separate from the casing (2).

15. Kit for carrying out a measurement analysis of the tear flow magnitude, comprising a compact device (1,1') according to any one of claims 5 to **14,** and a set of measurement strips (40) for carrying out the Schirmer test, intended for the diagnosis of ophthalmic diseases, especially for dry eye disease, said measuring strip (40) being especially suitable to be used to measure the magnitude of the maximum reflex tear secretion generated by the device (1, 1'), **characterized in that** the measurement strip is made up of a strip (40) of millimeter filter paper with a length greater than 30 mm, the proximal end of which is intended to be inserted into the space between the bulbar conjunctiva and the eyelid (41) as a tab with rounded edges to eliminate sharp angles and thus avoid irritation; and **in that** the filter paper strip (40) is sterilized and the tab (41) contains a dried solution of an ocular anesthetic; wherein the filter paper strip (40) has a width of approximately between 5 and 10 mm and a total length of approximately 50 mm.

## Patentansprüche

1. Verfahren zum Messen der Erzeugung einer maximalen Reflex-Tränensekretion, umfassend die chemische Stimulation der Hornhaut des Auges eines Individuums bestehend aus dem Anwenden auf die genannte Hornhaut eines kontrollierten Strahls einer Gasmischung mit einem hohen CO2-Gehalt, von mindestens 99 % CO2, um eine vorübergehende Verringerung beim pH des Tränenfilmes, welcher die Hornhaut deckt, hervorzubringen, aufgrund von der unmittelbaren Bildung von Kohlensäure, welche sich aus der Wechselwirkung in der Träne des CO2 mit dem H₂O der Träne ergibt, was zur Freisetzung von Protonen führt, welche die Nervenenden der polymodalen Nozizeptoren der Hornhautoberfläche stimulieren, wobei diese chemische Stimulation, mit der mechanischen und kalten Stimulation kombiniert, welche vom Gasstrahl evoziert wird, zur einer maximalen Reflex-Tränensekretion führt, deren Größe mittels herkömmlicher Techniken zur Tränenflussmessung gemessen werden kann, welche in Ophthalmologie und Optometrie zur Diagnose von Tränensekretionsstörungen von ophthalmologischen Erkrankungen, besonders für die Trockene-Augen-Erkrankung, verwendet werden, wobei der Gasstrahl oder -stoß auf die Hornhautoberfläche unter Aufrechterhaltung eines Trennabstandes gleich oder größer als 1 mm angewendet wird.

2. Verfahren zum Messen der Erzeugung einer Reflex-Tränensekretion nach Anspruch 1, **dadurch gekennzeichnet, dass** der Ausgangsdruck des Gasstrahls, welcher auf die Hornhautoberfläche angewendet wird, höher als der Atmosphärendruck ist.

3. Verfahren zum Messen der Erzeugung einer Reflex-Tränensekretion nach einem der vorhergehenden Ansprüche, **dadurch gekennzeichnet, dass** der Gasstrahl auf die Hornhautoberfläche mit einer Durchflussmenge gleich oder größer als 150 ml/Minute, vorzugsweise 200 ml/Minute, angewendet wird; und dass der Gasstrahl ein kurzfristiger Stoß ist, welcher in Richtung der Hornhautoberfläche eine vorgegebene Zeit lang angewendet wird, welche zwischen 2 und 6 Sekunden liegt, vorzugsweise von 4 Sekunden.

4. Verfahren zum Messen der Erzeugung einer Reflex-Tränensekretion nach einem der vorhergehenden Ansprüche, **dadurch gekennzeichnet, dass** der Gasstrahl oder -stoß auf die Hornhautoberfläche unter Aufrechterhaltung eines Trennabstandes gleich oder größer als 10 mm angewendet wird.

5. Kompakte Vorrichtung (1,1') zur Erzeugung einer Reflex-Tränensekretion, umfassend eine Gasquelle (7) in Form einer Einwegkartusche, welche ein Druckgas mit einer Zusammensetzung von mindestens 99 % CO2 enthält; und einen pneumatischen Kreislauf, welcher eine Ausgansmündung der genannten Gasquelle (7) mit einer Gasaustrittsdüse (3) der Vorrichtung (1,1') verbindet, wobei der Kreislauf Druckregelmittel (8) und Durchflussmengenregelmittel (17) umfasst, welche dafür geeignet sind, jeweils Gasstöße durch die Austrittsdüse (3) bei einem vorbestimmten Druck und vorbestimmten Durchflussmengen, zwischen 1 und 1,5 Bar und mindestens 150 ml/Minute, auszustoßen, wobei die Gasquelle (7) und der pneumatische Kreislauf in einem Gehäuse (2) zusammengebaut sind, welches mit einer Hand gegriffen werden kann oder an der Instrumentenhaltestange eines gewöhnlichen ophthalmischen Untersuchungstisches befestigt werden kann, und wobei die Vorrichtung (1,1') auch einen vom Benutzer bedienbaren Gasstoßauslöser (4) und einen Abstandshalter (5) mit einem proximalen Ende, welches mit dem Gehäuse (2) um die Austrittsdüse (3) herum gekoppelt ist, und einem distalen Ende, welches mit einem Umfangskante versehen ist, welche ergonomisch ausgebildet ist, um auf der Zielaugenhöhle eines Individuums gestützt zu werden, während die Vorrichtung (1.1') ausgelöst wird, umfasst, um einen Trennabstand zwischen der Gasaustrittsdüse (3) und der Hornhautoberfläche des Individuums bereitzustellen.

6. Kompakte Vorrichtung (1,1') nach Anspruch 5, **dadurch gekennzeichnet, dass** die Druckregelmittel (8) mit dem Ausgang der Gasquelle (7) eng verbunden sind und dazu ausgebildet sind, den Druck des Gasflusses am Ausgang der Gasquelle (7) auf einen vorher festgesetzten Sollwert, zwischen 1,2 und 1,6 Bar, zu regeln, um einen vorbestimmten Druck am Ausgang der Düse (3) von ungefähr zwischen 1,0 und 1,5, vorzugsweise 1,25 Bar, bereitzustellen.

7. Kompakte Vorrichtung (1,1') nach einem der Ansprüche 5 oder 6, **dadurch gekennzeichnet, dass** der pneumatische Kreislauf zusätzlich einen ersten Drucksensor (25), welcher dafür gestaltet ist, den Zwischendruck (P1) zu messen, welcher am Ausgang der Druckregelmittel (8) existiert; und einen zweiten Drucksensor (26), welcher dafür gestaltet ist, den Zwischendruck (P2) zu messen, welcher am Eingang zu den Durchflussmengenregelmitteln (17) existiert, umfasst, und dass der pneumatische Kreislauf auch Sicherheitsventilmittel (9,16) umfasst, welche dazu ausgebildet sind, die Abförderung des Gasflusses zu gewährleisten, wenn der von den Drucksensormitteln (25,26) detektierte Druck (P1, P2) höher als der eingestellte Druckwert an den jeweiligen Zwischenmesspunkten ist.

8. Kompakte Vorrichtung (1,1') nach einem der Ansprüche 5 bis 7, **dadurch gekennzeichnet, dass** sie Kontrollmittel umfasst, welche von einem bereitgestellten Mikroprozessor (27) einer geeigneten Software gesteuert werden, welcher dazu ausgebildet ist, die von den Drucksensoren (25,26), während einer aktivierten Auslösephase, detektierten Signale und das Signal des Ladezustands der elektrischen Zuführungsmittel (24) zu verarbeiten, zum Zwecke des Sendens eines Betätigungssignals zu einem Solenoidventil (13), welches stromabwärts der Drucksensoren (25,26) angeordnet ist, um durch die Düse (3) einen kontrollierten Gasstoß, das heißt, mit vorher festgesetzten Bedingungen von Ausgangsdruck und Fluss einer vorbestimmten Zeit lang, in Richtung der Hornhaut eines Individuums angewendet, aufzusprühen.

9. Kompakte Vorrichtung (1,1') nach dem vorhergehenden Anspruch, **dadurch gekennzeichnet, dass** der Mikroprozessor (27) mit einem persönlichen Kommunikationsnetzwerk (PAN) ausgerüstet ist, sodass es dessen Verbindung mit einer äußeren Vorrichtung erlaubt, welche in der Lage ist, Lese-/Schreibhandlugen über eine Schnittstelle/Anwendung durchzuführen, um mit der Identifizierung von Kartuschen assoziierte Information unter Verwendung einer NFC-Technologie zu erhalten.

10. Kompakte Vorrichtung (1,1') nach einem der Ansprüche 5 bis 9, **dadurch gekennzeichnet, dass** der Abstandshalter (5) asymmetrisch ist und um die Austrittsdüse (3) herum rotierbar ist, um in einem oder dem anderen Auge des Individuums untereinander austauschbar verwendet werden zu können.

11. Kompakte Vorrichtung (1,1') nach einem der Ansprüche 5 bis 9, **dadurch gekennzeichnet, dass** die Umfangskante des Abstandshalters (5), welche dazu vorgesehen ist, in Kontakt mit der Augenhöhle zu kommen, mittels eines Schutzelements (6) hergestellt aus einem biokompatiblen Material, wie Silikon, mit antibakteriellen und hypoallergenen Eigenschaften für eine größere Hygiene und Komfort, gedeckt ist.

12. Kompakte Vorrichtung (1,1') nach einem der Ansprüche 5 bis 11, **dadurch gekennzeichnet, dass** sie in Abmessungen geformt ist, sodass sie tragbar ist und mit einer Hand gegriffen werden kann, in Form einer Pistole oder ähnliches, wobei das Gehäuse (2) mit einem handhabeähnlichen Teil (2a) und einem Hauptkörper (2b) versehen ist, an welchem vorderen Ende die Austrittsdüse (3) der CO2-Stöße ist, und wobei der Auslöser (4) in einen Bereich des Gehäuses (2) integriert ist.

13. Kompakte Vorrichtung (1) nach Anspruch 12, **dadurch gekennzeichnet, dass** das Gehäuse (2) einen entfernbaren Teil umfasst, welcher sich im Bereich der Handhabe (2a) befindet, welcher mit einem inneren Hohlraum für das Platzieren einer CO2-Kartusche (7) versehen ist.

14. Kompakte Vorrichtung (1') nach einem der Ansprüche 5 bis 13, **dadurch gekennzeichnet, dass** sie derart geformt ist, dass sie mit der Instrumentenhaltestange eines gewöhnlichen ophthalmologischen Untersuchungstisches gekoppelt werden kann, und wobei der Auslöser Fernbetätigungsmittel umfasst, welche vom Gehäuse (2) getrennt sind.

15. Kit zum Durchführen einer Messanalyse der Größe des Tränenflusses, umfassend eine kompakte Vorrichtung (1,1') nach einem der Ansprüche 5 bis 14, und einen Satz von Messstreifen (40) zum Durchführen des Schirmer-Tests, welches zur Diagnose von ophthalmischen Erkrankungen, besonders für die Trockene-Augen-Erkrankung, vorgesehen ist, wobei der genannte Messstreifen (40) besonders geeignet zur Verwendung desselben bei der Messung der Größe der maximalen Reflex-Tränensekretion ist, welche von der Vorrichtung (1, 1') erzeugt wird, **dadurch gekennzeichnet, dass** der Messstreifen aus einem Streifen (40) aus Millimeterfilterpapier mit einer Länge größer als 30 mm besteht, dessen proximalen Ende dazu vorgesehen ist, in den Raum zwischen der Augenbindehaut und dem Augenlid (41) eingesetzt zu werden, als Lasche mit abgerundeten Kanten, um scharfe Winkel zu beseitigen und somit eine Reizung zu vermeiden; und dass der Filterpapierstreifen (40) sterilisiert ist und die Lasche (41) eine getrocknete Lösung eines Augenanästhetikums enthält; wobei der Filterpapierstreifen (40) eine Breite von ungefähr zwischen 5 und 10 mm und eine Gesamtlänge von ungefähr 50 mm aufweist.

## Revendications

1. Procédé pour mesurer la génération d'une sécrétion lacrymale réflexe maximale, comprenant la stimulation chimique de la cornée de l'œil de un sujet consistant à appliquer vers ladite cornée un jet contrôlé d'un mélange gazeux avec une teneur élevée en CO₂, d'au moins 99 % de CO₂, afin de produire une réduction transitoire du pH du film lacrymal qui recouvre la cornée, due à la formation immédiate d'acide carbonique, résultant de l'interaction dans les larmes du CO₂ avec l'H₂O des larmes, ce qui entraîne la libération de protons qui stimulent les terminaisons nerveuses des nocicepteurs polymodaux de la surface de la cornée, cette stimulation chimique, en combinaison avec la stimulation mécanique et par froid évoquée par le jet de gaz, entraîne une sécrétion lacrymale réflexe maximale, dont l'amplitude peut être mesurée par des techniques conventionnelles pour la mesure du flux de larmes utilisées en ophtalmologie et optométrie pour le diagnostic de troubles de sécrétion lacrymale de maladies ophtalmologiques, notamment pour la maladie de sécheresse oculaire, dans lequel le jet ou la bouffée de gaz est appliqué vers la surface de la cornée en maintenant une distance de séparation égale ou supérieure à 1 mm.

2. Procédé pour mesurer la génération d'une sécrétion lacrymale réflexe selon la revendication 1, **caractérisé en ce que** la pression de sortie du jet de gaz appliqué vers la surface de la cornée est supérieure à la pression atmosphérique.

3. Procédé pour mesurer la génération d'une sécrétion lacrymale réflexe selon l'une quelconque des revendications précédentes, **caractérisé en ce que** le jet de gaz est appliqué vers la surface de la cornée avec un débit égal ou supérieure à 150 ml/minute, de préférence 200 ml/minute; et **en ce que** le jet de gaz est une bouffée de courte durée appliquée en direction de la surface de la cornée pendant un temps prédéfini compris entre 2 et 6 seconds, de préférence 4 seconds.

4. Procédé pour mesurer la génération d'une sécrétion lacrymale réflexe selon l'une quelconque des revendications précédentes, **caractérisé en ce que** le jet ou la bouffée de gaz est appliqué vers la surface de la cornée en maintenant une distance de séparation égale ou supérieure à 10 mm.

5. Dispositif compact (1,1') for la génération d'une sécrétion lacrymale réflexe, comprenant une source de gaz (7) sous forme d'une cartouche de type jetable contenant un gaz sous pression avec une composition d'au moins 99 % de CO2; et un circuit pneumatique reliant une buse de sortie de ladite source de gaz (7) avec un embout de sortie de gaz (3) du dispositif (1,1'), le circuit comprenant des moyens de régulation de pression (8) et des moyens de régulation de débit (17) appropriés pour éjecter des bouffées du gaz à travers l'embout de sortie (3) à une pression et des débits prédéterminés, compris entre 1 et 1,5 bar et au moins 150 ml/minute, respectivement, la source de gaz (7) et le circuit pneumatique étant assemblés dans un boîtier (2) qui peut être saisi d'une seule main ou qui peut être attaché à la barre de support des instruments d'une table d'examen ophtalmique conventionnelle, et le dispositif (1,1') comprend également une gâchette de bouffée de gaz actionnable par l'utilisateur (4) et un support d'écartement (5) avec une extrémité proximale, couplée au logement (2) autour de l'embout de sortie (3), et une extrémité distale, pourvue d'un bord périphérique configuré de manière ergonomique pour être supporté sur l'orbite oculaire cible d'un sujet pendant le déclenchement du dispositif (1.1'), pour fournir une distance de séparation entre l'embout de sortie de gaz (3) et la surface de la cornée du sujet.

6. Dispositif compact (1,1') selon la revendication 5, **caractérisé en ce que** les moyens de régulation de pression (8) sont étroitement reliés à la sortie de la source de gaz (7) et configurés pour réguler la pression de l'écoulement de gaz à la sortie de la source de gaz (7) jusqu'à un point de consigne préétabli, comprise entre 1,2 et 1,6 bar, pour fournir une pression prédéterminée à la sortie de l'embout (3) comprise entre environ 1,0 et 1,5, de préférence 1,25 bar.

7. Dispositif compact (1,1') selon l'une quelconque des revendications 5 ou 6, **caractérisé en ce que** le circuit pneumatique comprend en outre un premier capteur de pression (25) conçu pour mesurer la pression intermédiaire (P1) existant à la sortie des moyens de régulation de pression (8); et un deuxième capteur de pression (26) conçu pour mesurer la pression intermédiaire (P2) existant à l'entrée dans les moyens de régulation de débit (17), et **en ce que** le circuit pneumatique comprend également des moyens de soupape de sécurité (9,16) configurés pour assurer l'évacuation de l'écoulement de gaz lorsque la pression (P1, P2) détectée par les capteurs de pression (25,26) est supérieure à la valeur de pression de consigne aux points de mesure intermédiaires respectifs.

8. Dispositif compact (1,1') selon l'une quelconque des revendications 5 à 7, **caractérisé en ce qu'**il comprend des moyens de contrôle gérés par un microprocesseur (27) pourvu d'un logiciel approprié, configuré pour traiter les signaux détectés par les capteurs de pression (25,26), pendant une phase de déclenchement activée, et le signal de l'état de charge de moyens d'alimentation électrique (24), afin d'envoyer un signal d'actionnement à une électrovanne (13) disposée en aval des capteurs de pression (25, 26) pour projeter à travers l'embout (3) une bouffée de gaz contrôlée, c'est-à-dire, avec des conditions préétablies de pression de sortie et d'écoulement pendant un temps prédéterminé appliquées dans la direction de la cornée d'un sujet.

9. Dispositif compact (1,1') selon la revendication précédente, **caractérisé en ce que** le microprocesseur (27) est pourvu de communications de zone personnelle (PAN), permettant la connexion avec un dispositif externe capable de réaliser des actions de lecture/écriture à travers une interface/application pour obtenir des informations associées à l'identification de cartouches en utilisant une technologie de NFC.

10. Dispositif compact (1,1') selon l'une quelconque des revendications 5 à 9, **caractérisé en ce que** le support d'écartement (5) est asymétrique et peut tourner autour de l'embout de sortie (3) pour pouvoir être utilisé de manière interchangeable dans un œil ou l'autre du sujet.

11. Dispositif compact (1,1') selon l'une quelconque des revendications 5 à 9, **caractérisé en ce que** le bord périphérique du support d'écartement (5) prévu pour entrer en contact avec l'orbite oculaire est recouvert par un élément de protection (6) réalisé en un matériau biocompatible, tel que la silicone, ayant des propriétés antibactériennes et hypoallergéniques pour une hygiène et confort supérieurs.

12. Dispositif compact (1,1') selon l'une quelconque des revendications 5 à 11, **caractérisé en ce qu'**il est conformé avec de dimensions telles qu'il est portable et peut être saisi d'une seule main, sous forme d'un pistolet ou similaire, dans lequel le boîtier (2) est pourvu d'une portion de type poignée (2a) et d'un corps principal (2b) à l'extrémité avant duquel se trouve l'embout de sortie (3) des bouffées de CO2, et la gâchette (4) étant intégrée dans une zone du logement (2).

13. Dispositif compact (1) selon la revendication 12, **caractérisé en ce que** le boîtier (2) comprend une portion amovible située dans la zone de la poignée (2a), pourvue d'une cavité interne pour le placement d'une cartouche de CO2 (7).

14. Dispositif compact (1') selon l'une quelconque des revendications 5 à 13, **caractérisé en ce qu'**il est conformé pour être couplé à la barre de support des instruments d'une table d'examen ophtalmologique conventionnelle, et dans lequel la gâchette comprend des moyens d'actionnement à distance séparés du boîtier (2).

15. Kit pour mettre en œuvre un analyse de mesure de l'amplitude du flux de larmes, comprenant un dispositif compact (1,1') selon l'une quelconque des revendications 5 à 14, et un ensemble de bandelettes de mesure (40) pour mettre en œuvre le test de Schirmer, prévu pour le diagnostic de maladies ophtalmiques, notamment pour la maladie de sécheresse oculaire, ladite bandelette de mesure (40) étant notamment appropriée pour être utilisée pour mesurer l'amplitude de la sécrétion lacrymale réflexe maximale générée par le dispositif (1, 1'), **caractérisé en ce que** la bandelette de mesure est constitué d'une bandelette (40) en papier filtre millimétré avec une longueur supérieure à 30 mm, dont l'extrémité proximale est prévue pour être insérée dans l'espace compris entre la conjonctive bulbaire et la paupière (41) comme une languette avec des bords arrondis pour éliminer des angles aigus et ainsi prévenir l'irritation; et **en ce que** la bandelette en papier filtre (40) est stérilisée et la languette (41) comporte une solution sèche d'un anesthésique oculaire; dans lequel la bandelette en papier filtre (40) présente une largeur comprise entre environ 5 et 10 mm et une longueur totale d'environ 50 mm.
